# EUROPEAN PATENT SPECIFICATION

(11) **EP 3 510 916 B1**
(45) Date of publication and mention of the grant of the patent: **25.01.2023**
(21) Application number: 18768518.5
(22) Date of filing: 12.03.2018
(51) Int. Cl.: A61B 1/12, A47L 17/08, B08B 9/057, A61B 90/70

(54) **TUBULAR MEDICAL DEVICE CLEANING BALL AND MANUFACTURING METHOD THEREFOR**
REINIGUNGSKUGEL FÜR EINE ROHRFÖRMIGE MEDIZINISCHE VORRICHTUNG UND HERSTELLUNGSVERFAHREN DAFÜR
BALLE DE NETTOYAGE DE DISPOSITIF MÉDICAL TUBULAIRE ET SON PROCÉDÉ DE FABRICATION

(30) Priority: 14.03.2017 KR 20170031584; 18.09.2017 KR 20170119287
(43) Date of publication of application: 17.07.2019
(73) Proprietor: Choi, Young Chul, Incheon 21698 (KR)
(72) Inventor: Choi, Young Chul, Incheon 21698 (KR)
(74) Representative: Garavelli, Paolo
(86) International application number: PCT/KR2018/002893
(87) International publication number: WO 2018/169268

(56) References cited:
- DE-A1-102006 035 258
- JP-A- 2004 350 966
- JP-U- H0 611 882
- KR-A- 20100 026 572
- KR-A- 20150 102 572
- KR-A- 20160 084 038
- KR-B1- 100 823 202
- US-A1- 2003 213 074
- US-A1- 2009 000 045

## Description

### Technical Field

The present invention relates to a cleaning ball for a tubular medical device and a method for manufacturing the same and, more particularly, a ball for cleaning a tubular medical device, which may be introduced together with a washing solution into the tubular medical device without using a separate brush and the like in order to clean the tubular medical device, which is inserted into a human body during minimally invasive surgery, thereby cleaning and sterilizing the tubular medical device, as well as a manufacturing method thereof.

### Background Art

In general, minimally invasive surgery is a surgical technique that minimizes incision in surgery wherein a surgical instrument is inserted through at least one small incision into the body of a patient (or animal to be subjected to surgery, etc.) to conduct the surgery.

A common form of such minimally invasive surgery may include endoscopic surgery and, in particular, laparoscopic surgery is the most common form of minimally invasive surgery wherein minimally invasive irradiation and surgical operation are conducted in the abdominal cavity.

In case of standard laparoscopic surgery, the abdomen of the patient is filled with gas and, after creating at least one small incision to provide an inlet for a laparoscopic surgical tool, a trocar is inserted through the inlet to thus perform the surgery.

During such surgical operation as described above, the user generally puts the laparoscopic surgical tool through the trocar into a surgical portion and then operates (or manipulates) the tool outside the abdominal cavity.

Meanwhile, the endoscope has been developed to be inserted into the body of a patient through natural orifice sites such as the mouth, anus or abdomen of the patient, thereby observing the state of affected parts of interest with the naked eye. This is a medical instrument necessary in health screening of a patient and having an important role.

As such, an endoscopic device inserted into a body organ or a catheter, trocar, etc. inserted in blood vessels is preferably used only one time without reusing the same, so as to prevent secondary infection possibly caused by reusing the above tool.

However, such a tubular medical device is mostly an expensive instrument and, if required according to hospital circumstances, is not disposed after a single use but is often reused after cleaning and sterilization.

On the other hand, although the surface of the tubular medical device is easily cleaned and sterilized while removing impurities adhered thereto in order to facilitate reuse thereof by any conventional disinfection or cleaning method, it is substantially difficult to clean and sterilize the inside of the tubular medical device.

Accordingly, in order to reuse the used medical instrument for examination of other patients, a technical solution for effectively sterilizing and removing pathogens, which is not harmful to the human body, such as in Korean Utility Model Laid-Open Publication No. 20-2016-0000822 (Patent Document 1), Korean Patent Laid-Open Publication No. 10-2013-0000043 (Patent Document 2), etc., has been continuously sought.

Conventional techniques for cleaning (disinfecting) a tubular medical device include, for example, sterilizing and disinfecting the tubular medical device with hot steam, applying ethylene oxide gas, heat drying, ultrasonic cleaning, and the like.

Further, in order to remove any residual material deposited on an inner surface of the tubular medical device, a cleaning method using a brush which can be inserted into a tubular device has been employed.

However, it is difficult to use the conventional cleaning method as described above if the medical device is sensitive to heat. Further, chemicals used in the conventional cleaning method may have adverse effects on the human body. Still further, the conventional cleaning method has a problem of not achieving sufficient cleaning effects.

In addition, the conventional method for cleaning a tubular medical device using a brush would necessarily leave behind blind spots which the brush cannot reach, depending upon a position and shape of the brush to be inserted into the tubular device. Further, there is a problem of not attaining satisfactory cleaning effects despite injecting a washing solution therein as a complementary measure.

Meanwhile, in order to solve the above conventional problems, there has been proposed a method for cleaning a tubular medical device wherein a number of cleaning balls with different sizes of several mm to several cm is introduced along with a washing solution such as water into the tubular medical device, thus cleaning the same. For example, document JP H06 11882 U (D1) discloses a cleaning ball for tubular medical devices made from two molded hemispherical caps.

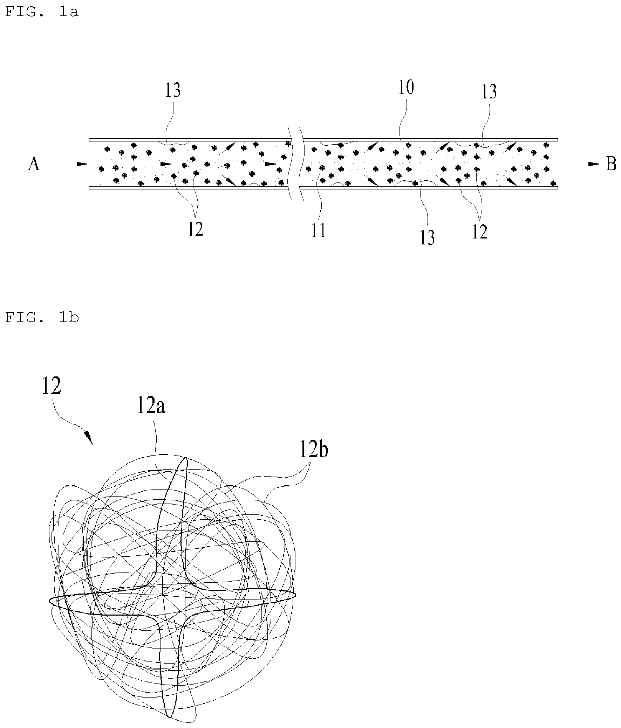

Referring to FIGS. 1(a) and 1(b), cleaning balls 12 as well as a washing solution 11 are introduced into a tubular medical device 10 requiring cleaning and, a residual material 13 deposited on an inner wall of the tubular medical device is scraped and removed during introduction (A) and discharge (B) of the cleaning balls 12.

Since the cleaning ball 12 has surface roughness, cleaning of the tubular medical device 10 using the cleaning ball could more easily and definitely remove the residual material 13 inside the medical device 10 without blind spots, compared to any conventional method.

The cleaning ball 12 is typically manufactured by winding a fiber, that is, a yarn around a bobbin 12a forming a framework while applying high pressure thereto, however, such a product is very difficult to manufacture.

Further, difficulty in manufacturing the cleaning ball 12 has led to a difficulty in mass-production of the same. Even when trying to prepare a ball of yarn by agglomerating simple fibers after removing the bobbin 12a, formation of the above product is not easy, causing a number of problems.

As such, use of the existing cleaning ball 12 described above is limited. Accordingly, there is currently a requirement for a novel cleaning ball 12 that can overcome the above problems, is easily mass-produced, and has excellent efficiency without using any typical brush.

### Disclosure

### Technical Problem

The present invention has been conceived to solve the foregoing problems and an object of the present invention is to provide a cleaning ball for a tubular medical device, which may be easily mass-produced by press-working an upper cap and a lower cap to form a ball, as well as a manufacturing method of the same.

Another object of the present invention is to provide a cleaning ball for a tubular medical device, which has surface roughness to thus completely remove irritating residues on the human body without blind spots inside the tubular medical device, as well as a manufacturing method of the same.

Further, another object of the present invention is to provide a cleaning ball for a tubular medical device, which includes a polymer or a polymer and an expanding agent in a hollow of the cleaning ball to increase a pressure applied to an inner wall of the tubular medical device while maintaining a spherical shape during cleaning, thereby maximizing cleaning effects, as well as a manufacturing method of the same.

A still further object of the present invention is to provide a cleaning ball for a tubular medical device, which can be easily mass-produced to thus reduce total costs, and may increase convenience in use, as well as a manufacturing method of the same.

### Technical Solution

In order to accomplish the above objects, the cleaning ball for a tubular medical device and the manufacturing method of the same according to the present invention will be described below.

The cleaning ball for a tubular medical device according to the present invention may be made of a textile fabric material having surface roughness, have a spherical shape with an internal hollow, and include a polymer inside the hollow, wherein the polymer has elasticity in order to maintain the spherical shape. The cleaning ball for a tubular medical device may include a hemispherical upper cap and a hemispherical lower cap that have the same size and shape wherein these caps are press-worked and integrally formed, and may have a flange protruded along a pressed bonding portion thereof.

If desired, the polymer may be surrounded by an expanding agent that absorbs water and is expanded.

Meanwhile, the polymer may include, for example, elastomer, sodium polyacrylate or polyacrylic acid ester.

The cleaning ball may be introduced together with a washing solution into the tubular medical device, and then discharged while scraping and removing residues deposited on an inner peripheral surface of the tubular medical device.

The cleaning ball may be expanded by the washing solution entering the hollow.

If desired, the upper cap and the lower cap may have different surface roughnesses, respectively.

A manufacturing method of the cleaning ball for a tubular medical device according to the present invention may include forming a cap type base, top and bottom alignment of the bases, and molding and cutting the same.

First, the cap type base formation step may include: placing a textile fabric with desired surface roughness and area between a top mold and a bottom mold wherein a recess part and a protrusion part correspondingly engaged with each other are formed in the top and bottom molds, respectively; press-working the textile fabric to form an upper cap base and a lower cap base, wherein each of the bases has a plurality of hemispheres formed on a surface thereof.

The top and bottom alignment of the upper and lower cap bases may include aligning the upper cap base and the lower cap base formed in the base formation step such that hemispheres correspond to each other in order to create a hollow in the center of the bases. Further, the top and bottom alignment step may further include placing a polymer in the hollow wherein the polymer has elasticity in order to maintain a spherical shape.

On the other hand, if desired, the top and bottom alignment step may be performed by providing an expanding agent layer, which is expanded by water absorption, in order to surround the polymer with the expanding agent.

Further, the molding and cutting step may include compressing the aligned upper cap base and lower cap base through a press and cutting the same, so as to manufacture a spherical shape cleaning ball having a hollow in the center thereof.

If desired, the molding and cutting step may further include an ultrasonic bonding process or a bonding process using an adhesive in order to bond the upper cap base and the lower cap base before compression and cutting.

Meanwhile, an upper cap formed of the hemispherical upper cap base and a lower cap formed of the hemispherical lower cap base may further include a flange protruded by compressing the bonded portion.

If desired, the upper cap and the lower cap may have different surface roughnesses, respectively.

### Advantageous effects

As described above, the cleaning ball for a tubular medical device and the manufacturing method of the same according to the present invention may be easily mass-produced by press-working a fabric having desired surface roughness in a spherical shape, reduce production and use costs, and improve convenience of use.

Further, the cleaning ball is made of a textile fabric material and has desired surface roughness, and therefore, may completely remove irritating residues on the human body without damage to the tubular medical device and blind spots inside the same during cleaning.

In addition, when the tubular medical device is cleaned by a polymer or a polymer and an expanding agent included in a hollow of the cleaning ball, the cleaning ball may maintain the spherical shape regardless of external pressure. Further, the cleaning ball is expanded by absorbed water to thus increase pressure applied to an inner wall of the tubular medical device, thereby maximizing cleaning efficiency during cleaning.

### Description of Drawings

FIG. 1a is a schematic view illustrating cleaning of a tubular medical device according to the conventional art.
FIG. 1b is a schematic view showing the cleaning ball for a tubular medical device employed in the conventional art.
FIG. 2 is a perspective view showing the cleaning ball for a tubular medical device according to the present invention.
FIG. 3 is a cross-sectional view showing the cleaning ball for a tubular medical device according to an embodiment of the present invention.
FIGS. 4 to 6 are schematic views and a flowchart illustrating a manufacturing method of the cleaning ball for a tubular medical device according to the present invention.
FIG. 7 is a schematic view illustrating a state of use of the cleaning ball for a tubular medical device according to the present invention.

### Best Mode

Hereinafter, preferred embodiments of the cleaning ball for a tubular medical device and the manufacturing method of the same according to the present invention will be described in detail with reference to the accompanying drawings.

Then, a detailed description of known functions and configurations in relation to the following description of the present invention will be omitted to avoid obscuring the subject matter of the present invention.

In addition, a tubular medical device 10 shown in the drawings has an inlet A and an outlet B which are located at different sites. However, this is intended as only one example for convenience of description and the tubular medical device 10 may of course have the inlet A and the outlet B at the same position, depending upon a shape of the tubular medical device.

Further, with regard to the description of the present invention, the same elements as described in the conventional art will be defined and explained to have the same reference numerals.

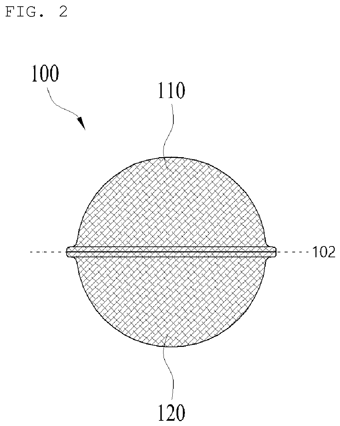

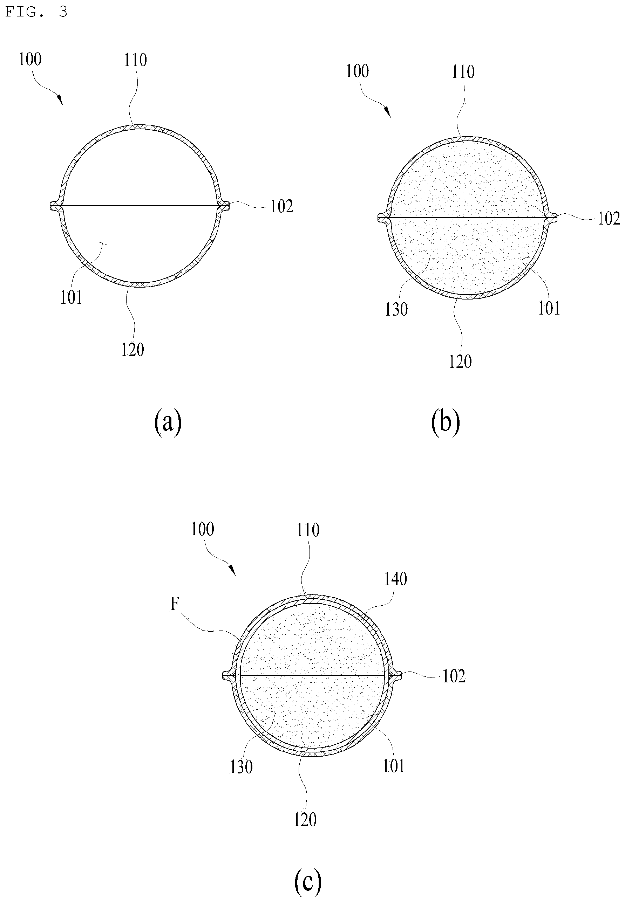

First, FIG. 2 is a perspective view showing the cleaning ball for a tubular medical device according to the present invention, while FIG. 3 is a cross-sectional view showing the cleaning ball for a tubular medical device according to an embodiment of the present invention.

As shown in the drawings, the cleaning ball 100 for a tubular medical device according to the present invention is made of a textile fabric material having surface roughness and has a spherical shape including a hollow 101 therein.

The cleaning ball 100 is introduced together with a washing solution 11 into a tubular medical device 10 and then is discharged to the outside while scraping and removing residues 13 deposited on an inner peripheral surface of the tubular medical device 10.

More particularly, the cleaning ball 100 is made of a textile fabric material having surface roughness, and is integrally formed by press-working a hemispherical upper cap 110 and a hemispherical lower cap 120, which have the same size and shape, thus resulting in a spherical shape including a hollow 101 therein.

Further, the pressed portions of the upper cap 110 and the lower cap 120 form a protruding flange 102, wherein the flange 102 may play a role of more easily scraping an inner wall of the tubular medical device 10 so as to remove the residues 13.

Referring to FIG. 3 (a), the cleaning ball 100 for a tubular medical device may be formed as a pure empty space wherein a polymer 130 and an expanding agent 140 are not included in the hollow 101.

The cleaning ball 100 is introduced together with a washing solution into the tubular medical device 10, wherein the washing solution 11 penetrates into the hollow 101 in accordance with material properties of the textile fabric forming the surface of the cleaning ball, thereby expanding the surface of the cleaning ball 100 and increasing a surface area thereof.

In this case, the washing solution 11 injected and penetrated into the hollow 101 of the cleaning ball 100 has an internal pressure responding to the washing solution outside the cleaning ball, and therefore, the expanded cleaning ball 100 may have an internal pressure responding more or less to an external pressure and an original shape of the cleaning ball 100 may be maintained. Accordingly, the expanded cleaning ball 100 can scrape the inner wall of the tubular medical device due to an increase in surface area, thereby increasing cleaning efficiency.

Herein, the upper cap 110 and the lower cap 120 have the same surface roughness. However, if necessary, these caps may be formed to have different surface roughnesses using materials having different roughnesses, respectively.

Referring to FIG. 3(b), the cleaning ball 100 may include a polymer 101a having elasticity in the hollow 101, so as to maintain the original shape thereof regardless of an external pressure when the washing solution is injected into the tubular medical device 10 for cleaning the inside of the same.

In this case, the polymer 101a fills the hollow 101 and may be present in the form of a single polymer or multiple polymers.

According to one example, the polymer 101a used herein is usually silicone or rubber and, more particularly, elastomer, sodium polyacrylate or polyacrylic acid ester.

Such polymer 101a may have inherent elasticity to maintain an original shape thereof or, when the washing solution 11 penetrates into the hollow 101, may absorb the washing solution to thus be expanded or gelled, thereby enabling the cleaning ball 100 to maintain a ball shape regardless of external pressure. On the other hand, the polymer 130 used herein may be a polymer without water absorption and expansion in consideration of production cost and ease of manufacturing.

Next, referring to FIG. 3(c), if desired, the cleaning ball 100 for a tubular medical device according to another example may comprise the polymer 130 surrounded by an expanding agent 140 which absorbs water and is expanded.

Preferably, the expanding agent 140 has a higher coefficient of expansion than that of the polymer 130 and may include a material that can sufficiently absorb water and then be expanded.

During cleaning of the tubular medical device 10, the upper cap 11 and the lower cap 120 forming a surface of the cleaning ball 100 may have an empty space generated between the polymer 130 and the upper cap 110 or the lower cap 120 when the textile fabric absorbs the washing solution and is expanded. In this case, it may be concerned that the space is distorted by external pressure.

However, if the expanding agent 140 is included, the above problem may be overcome. Accordingly, the expanding agent 140 may comprise a material having a high coefficient of expansion for expansion by water absorption.

As such, owing to a configuration that is integrally formed by surrounding an outer part of the polymer 130 with the expanding agent 140, the washing solution 11 injected during cleaning of the tubular medical device may be absorbed not only in the textile fabric forming an outer surface of the cleaning ball 100 but also in the expanding agent 140.

The expanding agent 140 may be expanded while absorbing the washing solution, and then, further expand the upper cap 110 and the lower cap 120 so as to increase an outer surface area. In addition, the expanding agent may endure an external pressure along with the internal polymer 130, thereby facilitating the cleaning ball 110 to maintain a spherical shape.

Such an expanding agent 140 used herein is preferably agar using Ceylon moss (or agar-agar) and, if it is a material having a high coefficient of expansion for expansion by water absorption, various materials other than the aforementioned one may of course be used.

If the polymer 130 and the expanding agent 140 are included in the hollow 101 of the cleaning ball 100 according to the present invention, the cleaning ball 100 may have a coefficient of volume expansion due to absorption of the washing solution 11 in a range of 15 to 35% or 20 to 30%.

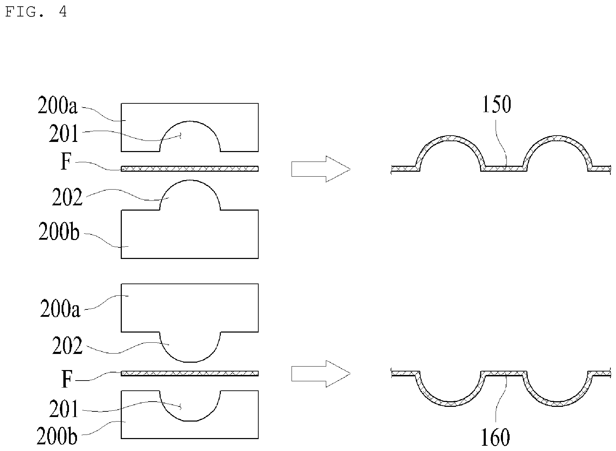

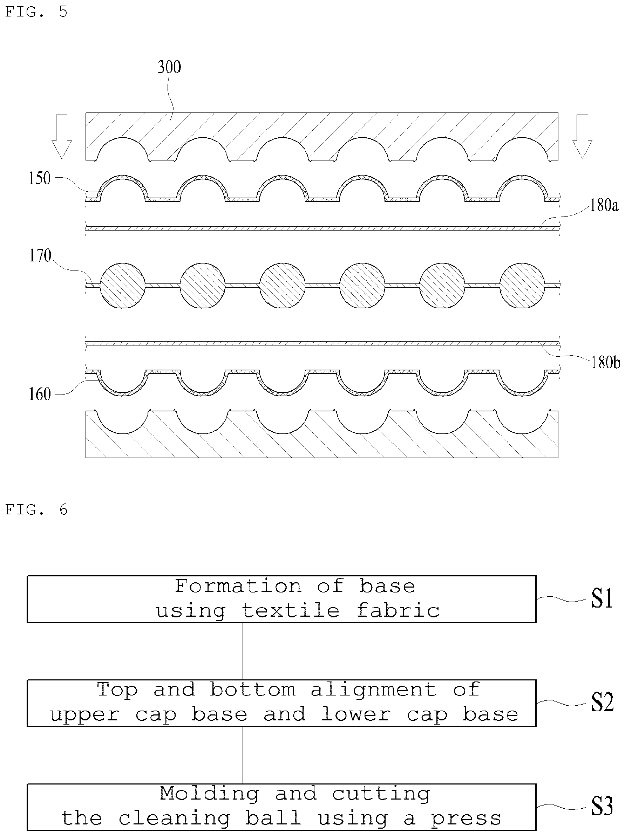

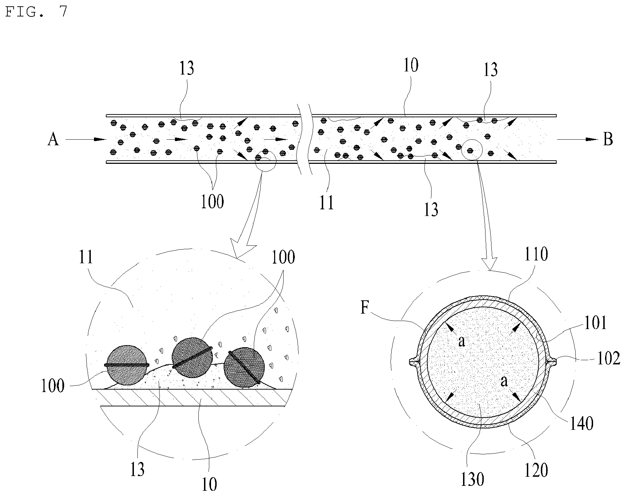

Next, a manufacturing method of the cleaning ball for a tubular medical device according to the present invention will be described in detail. FIGS. 4 to 6 are schematic views and a flowchart illustrating the manufacturing method of the cleaning ball for a tubular medical device according to the present invention, while FIG. 7 is a schematic view illustrating a state of use of the cleaning ball for a tubular medical device according to the present invention.

As described above, the cleaning ball 100 for a tubular medical device according to the present invention is made of a textile fabric material, and is integrally formed by closely contacting the upper cap 110 with the lower cap 120 and press-working the same, thereby being formed to have an overall spherical shape.

More specifically, the cleaning ball 100 may be manufactured into a finished product by a base formation step S1, a top and bottom alignment step S2 and a molding and cutting step S3.

First, the base formation step S1 may be conducted to prepare hemispherical shapes of the upper cap 110 and the lower cap 120 forming the cleaning ball 100, and may include forming an upper cap base 150 and a lower cap base 160 wherein these bases include a plurality of the upper caps 110 and lower caps 120 in a cap form.

For this purpose, the manufacturing method may include: placing a textile fabric F with desired surface roughness and area between a top mold 200a and a bottom mold 200b wherein a recess part 201 and a protrusion part 202 correspondingly engaged with each other are formed in the top and bottom molds, respectively; and press-working the textile fabric F to form an upper cap base 150 and a lower cap base 160, wherein each of the bases has a plurality of hemispheres formed on a surface thereof.

According to the aforementioned processes, the textile fabric F in a flat shape is reconstituted into the upper cap base 150 and the lower cap base 160 that have protruding embossing faces.

Herein, both of the upper cap base 150 and the lower cap base 160 may be prepared in the same mold or, if necessary, may of course be prepared in a separate mold.

On the other hand, the textile fabric F has a size and an area sufficient to fully cover a molding face of the top mold 200a and the bottom mold 200b, and may have as many hemispheres, that is, embossed portions, as the number of recessed parts 201 and protrusion parts 202 present in the top mold and the bottom mold, respectively.

Preferably, the top mold 200a and the bottom mold 200b have multiple recessed parts 201 only or multiple protrusion parts 202 only, along the overall molding face.

Next, the top and bottom alignment step S2 may include aligning the upper cap base 150 and the lower cap base 160 formed in the base formation step S1 such that hemispheres correspond to each other in order to create a hollow 101 in the center of the bases.

As a result, the upper cap base 150 and the lower cap base 160 may have a cleaning ball shape 100 that has the hollow 101 while facing each other.

When the cleaning ball 100 is manufactured through press-working under conditions as described above, a cleaning ball having an empty hollow space 101 as shown in FIG. 3(a) is produced.

Further, in order to manufacture the cleaning ball 100 as shown in FIG. 3(b), the top and bottom alignment step S2 may include positioning a polymer layer 170 such that the polymer 130 having elasticity is included in the hollow 101 in order to maintain the spherical shape.

Herein, the polymer 130 may be present in the form of a single polymer or multiple polymers so as to fill the hollow 101.

The drawings have illustrated that the polymer 130 could be previously prepared and used in an embossing form having a size with which the polymer 130 can be included in the hollow 101 formed by the upper cap base 150 and the lower cap base 160, without being limited thereto. If necessary, a plate type polymer layer 170 having a desired thickness may of course be prepared in a mold by compression.

Further, a method for preparation of the polymer 130 is not particularly limited. In the case of silicone, the polymer 130 may of course be prepared by injecting the silicone into the hollow 101 by means of a gun type injector.

On the other hand, the polymer 130 may have inherent elasticity to maintain an original shape thereof. Otherwise, since the washing solution 11 penetrates into the hollow 101, the polymer may absorb the washing solution and thus be expanded or gelled, thereby facilitating the cleaning ball 100 to maintain a ball shape regardless of external pressure.

As described above, maintaining the shape of the cleaning ball 100 by the polymer 130 may maximize cleaning efficiency by sufficiently scraping and removing residues out of the tubular medical device 10 while passing through the inside of the tubular medical device 10. Types of the polymer may include, for example, elastomer, sodium polyacrylate or polyacrylic acid ester, as described above.

Further, in order to manufacture the cleaning ball 100 as shown in FIG. 3(c), the top and bottom alignment step S2 may include placing an expanding agent layer 180a or 180b, which is made of a material expanded by water absorption, on the top and bottom of the polymer layer 170 to surround the polymer 130 with the expanding agent 140.

Accordingly, the formed expanding agent 140 may absorb the washing solution and then be expanded to endure an external pressure along with the internal polymer 130, thereby helping the cleaning ball 100 to maintain a spherical shape.

According to one example, the expanding agent layer 180a or 180b used herein may be agar using agar-agar weeds, without being limited thereto. A variety of materials may of course be applied as long as it is a material having a high coefficient of expansion for expansion by water absorption.

The cleaning ball 100 manufactured by the above method, which includes placing the expanding agent layer in a desired position, may include the polymer 130 and the expanding agent 140 in the hollow 101.

Subsequently, the molding and cutting step S3 may include compressing the upper cap base 150 and the lower cap base 160, which were aligned in the top and bottom alignment step S2, through a press 300 and cutting the same, thereby manufacturing a spherical shape cleaning ball 100 having a hollow 101 in the center thereof.

The textile fabrics F forming the upper cap base 150 and the lower cap base 160 are bonded to each other by pressure and heat generated during press-working, and the hemispheres facing each other may form a hollow 101 in the center thereof and produce a single cleaning ball 100.

Further, according to a size of the textile fabric F having the above area and the number of hemispheres, the cleaning ball 100 may be manufactured in plural at the same time.

On the other hand, if necessary, the molding and cutting step S3 may of course include a separate ultrasonic bonding process and a bonding process using an adhesive at a bonding surface in order to bond the upper cap base 150 and the lower cap base 160 before the cutting process.

Referring to FIG. 7, a state of use of the cleaning ball 100 for a tubular medical device according to the present invention described above will be reviewed as follows.

In this regard, the cleaning ball 100 having a hollow 101 filled with the polymer 130 and the expanding agent 140 will be explained for illustrative purpose.

A tubular medical device 10 used for operation requires cleaning and sterilization depending upon use thereof, and may include residues 13 therein.

In order to clean the tubular medical device 10, the tubular medical device 10 may be subjected to ultrasonic cleaning or washing by immersing the same in a washing container. Further, in order to completely clean and sterilize the inside of the tubular medical device 10, a washing solution 11 is injected into the tubular medical device 10 through an inlet A and then is discharged through an outlet B, thereby cleaning the same.

At this time, the washing solution 11 used herein may be saline, water or water containing medicaments for disinfection, without being particularly limited to any one thereof.

Further, in addition to the washing solution 11 injected through the inlet A, if required, a number of cleaning balls 100 may also be introduced by adding the same to the washing solution.

The cleaning ball 100 is preferably pre-introduced in a state of being included in the washing solution 11 and, if desired, may of course be introduced separately independently of the washing solution 11.

As described above, the cleaning ball 100 is preferably made of a textile fabric F, may have surface roughness and be expandable by penetration of the washing solution 11 into the hollow 101.

Then, at a bonding site between the upper cap 110 and the lower cap 120, a flange 102 protruded by press-working is included. Therefore, the flange 102 as well as a rough potion of the surface of the cleaning ball 100 are introduced into the tubular medical device 10 and then discharged while scraping and removing residues 13 deposited or remaining on an inner wall of the tubular medical device when moving from the inlet A to the outlet B.

In this case, the cleaning ball 100 can have sufficient strength to maintain the original shape thereof by the washing solution 11 penetrated into the hollow 101 regardless of external pressure, and the polymer 130 included in the hollow has elasticity to thus help the cleaning ball 100 more easily maintain a spherical shape.

Specifically, in case of a structure including the expanding agent 140, the expanding agent 140 is a material having a high coefficient of expansion for expansion by water absorption, may compress the textile fabric F from the inside to the outside of the cleaning ball, thereby helping the cleaning ball 100 more easily maintain a spherical shape regardless of an external pressure, thus further maximizing cleaning efficiency.

The cleaning ball 100 configured as described above may more easily remove residues 13 while moving inside the tubular medical device 10.

Further, the washing solution 11 may form a vortex in accordance with a shape of the tubular medical device 10 or an injection pressure of the washing solution while being injected into the tubular medical device 10, wherein the cleaning ball 100 may perform cleaning while hitting the inner wall of the tubular medical device 10.

Meanwhile, the cleaning ball 100 may have a size in a range of several mm to several cm depending upon the shape and size of the tubular medical device 10. Although not shown in the drawings, the cleaning ball may be introduced in desired quantity (or numbers) sufficient to fill the entirety of the inner space of the tubular according to the cleaning method. According to one example, a size of the cleaning ball 100 may range from 2 mm to 5 mm according to use thereof, without being particularly limited thereto.

In this regard, the cleaning ball 100 is expanded and has increased surface area due to penetration of the washing solution 11 into the hollow 101, and may be positioned while fully filling the inner space of the tubular medical device 10.

Further, the cleaning ball 100 expanded by a pressure of the injected washing solution 11 may move along an inner peripheral surface of the tubular medical device 10 and thus more completely clean the inner wall of the tubular medical device 10.

In other words, with regard to the cleaning ball 100, the number of introduced cleaning balls may be selected depending upon the shape of the tubular medical device 10. Further, the cleaning ball may be introduced together with the washing solution 11, or otherwise, may be introduced separately.

Therefore, as described above, the cleaning ball 100 for a tubular medical device according to the present invention may completely remove irritating residues on the human body without blind spots inside the tubular medical device.

With respect to the present invention as described above, various substitutions, modifications and alterations may be possible by persons having ordinary skill in the art, to which the present invention pertains, without departing from the scope of the present invention. Therefore, the present invention is duly not limited to the aforementioned embodiments and accompanying drawings.

## Claims

1. A cleaning ball configured to be introduced together with a washing solution (11) into a tubular medical device (10) and then to be discharged while scraping and removing residues (13) deposited on an inner peripheral surface of the tubular medical device (10), the cleaning ball comprising:
a hemispherical upper cap (110) and a hemispherical lower cap (120), which are made of a textile fabric material having surface roughness and have the same size and shape to form a spherical shape having a hollow (101) therein, wherein the upper cap and the lower cap are press-worked and integrally formed, and have a flange (102) protruding along the pressed bonding portion.

2. The cleaning ball according to claim 1, wherein the hollow (101) includes a polymer (130) having elasticity so as to maintain a ball shape inside the hollow.

3. The cleaning ball according to claim 2, wherein the polymer (130) is any one of elastomer, sodium polyacrylate or polyacrylic acid ester.

4. The cleaning ball according to claim 2, wherein the polymer (130) is surrounded by an expanding agent (140) that absorbs water and is expanded.

5. The cleaning ball according to claim 1, wherein the upper cap (110) and the lower cap (120) have different surface roughnesses, respectively.

6. A method of manufacturing a cleaning ball according to claim 1, the method
comprising:
a cap type base formation step (S1) which includes: placing a textile fabric (F) having desired surface roughness and area between a top mold (200a) and a bottom mold (200b), wherein a recessed part (201) and a protrusion part (202) correspondingly engaged with each other are formed in the top and bottom molds, respectively; and press-working the textile fabric (F) to form an upper cap base (150) and a lower cap base (160), wherein each of the bases has a plurality of hemispheres formed on a surface thereof;
a top and bottom alignment step (S2) which includes aligning the upper cap base (150) and the lower cap base (160) formed in the base formation step (S1) such that hemispheres correspond to each other to form a hollow (101) in the center of the bases; and
a molding and cutting step (S3) which includes compressing the upper cap base (150) and the lower cap base (160) aligned in the top and bottom alignment step (S2) through a press (300) and cutting the same, thereby manufacturing the cleaning ball (100) in a spherical shape which includes a hollow (101) in the center thereof.

7. The method according to claim 6, wherein the top and bottom alignment step (S2) includes positioning a polymer layer (170) such that a polymer (130) having elasticity is included in the hollow (101) so as to maintain the spherical shape.

8. The method according to claim 7, wherein the top and bottom alignment step (S2) includes positioning an expanding agent layer (180a) or (180b), which is made of a material expanded by water absorption, so as to surround the polymer (130) with an expanding agent (140).

9. The method according to claim 6, wherein the molding and cutting step (S3) includes an ultrasonic bonding process or a bonding process using an adhesive in order to bond the upper cap base (150) and the lower cap base (160) before compressing and cutting.

## Patentansprüche

1. Reinigungskugel, welche so konfiguriert ist, dass sie zusammen mit einer Waschlösung (11) in eine röhrenförmige medizinische Vorrichtung (10) eingeführt und dann ausgetragen wird, während sie Rückstände (13), welche sich auf einer inneren Umfangsfläche der röhrenförmigen medizinischen Vorrichtung (10) abgelagert haben, abschabt und entfernt, wobei die Reinigungskugel umfasst:
eine halbkugelförmige obere Kappe (110) und eine halbkugelförmige untere Kappe (120), welche aus einem textilen Gewebematerial mit Oberflächenrauheit hergestellt sind und die gleiche Größe und Form aufweisen, um eine Kugelform mit einem Hohlraum (101) darin zu bilden, wobei die obere Kappe und die untere Kappe pressgeformt und einstückig ausgebildet sind und einen Flansch (102) aufweisen, welcher entlang des gepressten Verbindungsabschnitts vorsteht.

2. Reinigungskugel nach Anspruch 1, wobei der Hohlraum (101) ein Polymer (130), welches eine Elastizität zur Aufrechterhaltung einer Kugelform im Inneren des Hohlraums aufweist, enthält.

3. Reinigungskugel nach Anspruch 2, wobei das Polymer (130) eines der folgenden ist: Elastomer, Natriumpolyacrylat oder Polyacrylsäureester.

4. Reinigungskugel nach Anspruch 2, wobei das Polymer (130) von einem Expansionsmittel (140) umgeben ist, welches Wasser absorbiert und sich ausdehnt.

5. Reinigungskugel nach Anspruch 1, wobei die obere Kappe (110) und die untere Kappe (120) jeweils unterschiedliche Oberflächenrauheiten aufweisen.

6. Verfahren zur Herstellung einer Reinigungskugel nach Anspruch 1, wobei das Verfahren umfasst:
einen Schritt (S 1) zur Bildung einer kappenartigen Basis, welcher umfasst: Anordnen eines Textilgewebes (F) mit einer gewünschten Oberflächenrauheit und -größe zwischen einer oberen Form (200a) und einer unteren Form (200b), wobei ein ausgesparter Teil (201) und ein vorstehender Teil (202), die entsprechend miteinander in Eingriff stehen, in der oberen bzw. unteren Form gebildet werden; und
Pressformen des Textilgewebes (F), um eine obere Kappenbasis (150) und eine untere Kappenbasis (160) zu bilden, wobei jede der Basen mehrere Halbkugeln aufweist, welche auf deren Oberfläche gebildet sind;
einen Ausrichtungsschritt (S2) für die Ober- und Unterseite, welcher das Ausrichten der oberen Kappenbasis (150) und der unteren Kappenbasis (160), welche in dem Schritt der Basisbildung (S1) erzeugt wurden, umfasst, so dass die Halbkugeln sich decken, um einen Hohlraum (101) in der Mitte der Basen zu bilden; und
einen Form- und Schneideschritt (S3), welcher das Zusammendrücken der oberen Kappenbasis (150) und der unteren Kappenbasis (160), welche in dem Ausrichtungsschritt (S2) der Ober- und Unterseite ausgerichtet wurden, durch eine Presse (300) und das Schneiden derselben umfasst, wodurch die Reinigungskugel (100) in einer Kugelform hergestellt wird, welche einen Hohlraum (101) in ihrer Mitte umfasst.

7. Verfahren nach Anspruch 6, wobei der Ausrichtungsschritt (S2) für die Ober- und Unterseite das Positionieren einer Polymerschicht (170) umfasst, so dass ein Polymer (130), welches eine Elastizität aufweist, in den Hohlraum (101) eingeschlossen wird, um die Kugelform zu erhalten.

8. Verfahren nach Anspruch 7, wobei der Ausrichtungsschritt (S2) für die Ober- und Unterseite das Positionieren einer Expansionsmittelschicht (180a) oder (180b) umfasst, welche aus einem durch Wasserabsorption expandierten Material hergestellt ist, um das Polymer (130) mit einem Expansionsmittel (140) zu umgeben.

9. Verfahren nach Anspruch 6, wobei der Form- und Schneideschritt (S3) einen Ultraschallverbindungsprozess oder einen Verbindungsprozess unter Verwendung eines Klebstoffs umfasst, um die obere Kappenbasis (150) und die untere Kappenbasis (160) vor dem Zusammendrücken und Schneiden zu verbinden.

## Revendications

1. Balle de nettoyage configurée pour être introduite avec une solution de lavage (11) dans un dispositif médical tubulaire (10), puis pour être déchargée tout en raclant et en enlevant les résidus (13) déposés sur une surface périphérique interne du dispositif médical tubulaire (10), la balle de nettoyage comprenant :
un capuchon supérieur hémisphérique (110) et un capuchon inférieur hémisphérique (120), qui sont faits d'un matériau de tissu textile ayant une rugosité de surface et ont la même taille et la même forme pour former une forme sphérique ayant un creux (101) à l'intérieur, dans lequel le capuchon supérieur et le capuchon inférieur sont moulés à la presse et formés d'un seul tenant, et ont une bride (102) faisant saillie le long de la partie de liaison pressée.

2. Balle de nettoyage selon la revendication 1, dans laquelle le creux (101) comprend un polymère (130) ayant une élasticité de manière à maintenir une forme de balle à l'intérieur du creux.

3. Balle de nettoyage selon la revendication 2, dans laquelle le polymère (130) est l'un quelconque parmi un élastomère, un polyacrylate de sodium ou un ester d'acide polyacrylique.

4. Balle de nettoyage selon la revendication 2, dans laquelle le polymère (130) est entouré d'un agent d'expansion (140) qui absorbe l'eau et s'élargit.

5. Balle de nettoyage selon la revendication 1, dans laquelle le capuchon supérieur (110) et le capuchon inférieur (120) ont des rugosités de surface différentes, respectivement.

6. Procédé de fabrication d'une balle de nettoyage selon la revendication 1, le procédé comprenant :
une étape de formation de socle de type capuchon (S1) qui comprend :
le placement d'un tissu textile (F) ayant une rugosité de surface et une zone souhaitées entre un moule supérieur (200a) et un moule inférieur (200b), dans lequel une partie en retrait (201) et une partie en saillie (202) engagées de manière correspondante l'une avec l'autre sont formées dans les moules supérieur et inférieur, respectivement ; et le moulage à la presse du tissu textile (F) pour former un socle de capuchon supérieur (150) et un socle de capuchon inférieur (160), dans lequel chacun des socles a une pluralité d'hémisphères formés sur une surface de celui-ci ;
une étape d'alignement supérieur et inférieur (S2) qui comprend l'alignement du socle de capuchon supérieur (150) et du socle de capuchon inférieur (160) formés dans l'étape de formation de socle (S1) de sorte que les hémisphères correspondent les uns aux autres pour former un creux (101) au centre des socles ; et
une étape de façonnage et de découpe (S3) qui comprend la compression du socle de capuchon supérieur (150) et du socle de capuchon inférieur (160) alignés dans l'étape d'alignement supérieur et inférieur (S2) à travers une presse (300) et la découpe de ceux-ci, fabriquant ainsi la balle de nettoyage (100) sous une forme sphérique qui comprend un creux (101) en son centre.

7. Procédé selon la revendication 6, dans lequel l'étape d'alignement supérieur et inférieur (S2) comprend le positionnement d'une couche de polymère (170) de telle sorte qu'un polymère (130) présentant une élasticité est inclus dans le creux (101) de manière à maintenir la forme sphérique.

8. Procédé selon la revendication 7, dans lequel l'étape d'alignement supérieur et inférieur (S2) comprend le positionnement d'une couche d'agent d'expansion (180a) ou (180b), qui est faite d'un matériau élargi par absorption d'eau, de façon à entourer le polymère (130) avec un agent d'expansion (140).

9. Procédé selon la revendication 6, dans lequel l'étape de façonnage et de découpe (S3) comprend un procédé de liaison par ultrasons ou un procédé de liaison utilisant un adhésif afin de lier le socle supérieur de capuchon (150) et le socle inférieur de capuchon (160) avant la compression et la découpe.
